# EUROPEAN PATENT APPLICATION

(11) **EP 0 776 905 A2**
(43) Date of publication of application: **04.06.1997**
(21) Application number: 96308525.3
(22) Date of filing: 26.11.1996
(51) Int. Cl.: C07J 17/00, C07J 9/00, C07J 31/00

(54) **Process for the preparation of (24R)-24,25-dihydroxysteroid compounds**

(30) Priority: 27.11.1995 JP 331042/95; 16.02.1996 JP 54015/96
(71) Applicant: KUREHA KAGAKU KOGYO KABUSHIKI KAISHA, Chuo-ku Tokyo 103 (JP)
(72) Inventor: Konai, Yutaka, Machida-shi, Tokyo (JP); Otomo, Mituru, Iwaki-shi, Fukushima-ken (JP); Katsurao, Takumi, Iwaki-shi, Fukushima-ken (JP)
(74) Representative: Woods, Geoffrey Corlett

(57) **Abstract**

A process for producing a (24R)-24,25-dihydroxysteroid compound which comprises carrying out a catalytic asymmetric dihydroxylation reaction on a steroid compound having a carbon-carbon double bond in the side chain in the presence of an osmium complex having a hydroquinidine derivative using a heterogeneous solvent system prepared by adding at least one organic solvent selected from tetrahydrofuran, acetonitrile, acetone, 1,4-dioxane, N,N-dimethylformamide and N,N-dimethylacetamide to a tert-butanol/water two-component solvent system.

## Description

The present invention relates to a process for the preparation of (24R)-24,25-dihydroxysteroid compounds, and more particularly, relates to a process for the selective preparation of (24R)-diastereomers.

The following techniques are known for the preparation of (24R)-24,25-dihydroxyvitamin D₃ using a (24R)-24,25-dihydroxycholesterol derivative as an intermediate. Firstly, 5β-cholest-24-ene-3α,6α-diol is subjected successively to the treatments of tosylation, epoxidation, acid catalytic hydrolysis for the epoxide, reaction with potassium acetate and saponification to produce (24RS)-24,25-dihydroxycholesterol (Japanese Patent Publication (KOKOKU) No. 4-57679).

This compound is converted into a derivative from which diastereomers can be easily separated, thus a (24R)-24,25-dihydroxycholesterol derivative is separated and used as an intermediate for (24R)-24,25-dihydroxyvitamin D₃ (Japanese Patent Publication (KOKOKU) No. 1-49279, etc.).

As described above, a multistage process is required for synthesizing a (24R)-24,25-dihydroxycholesterol derivative, and further, in the course of the synthesis, the 24R-form and 24S-form thereof are produced as a substantially equivalent mixture.

It is also known that (24RS)-24,25-dihydroxycholesterol derivatives can be obtained by subjecting desmosterol derivatives to epoxidation and acid catalytic hydrolysis successively or by dihydroxylating them with osmium tetroxide and converting the resulting products into (24R)-24,25-dihydroxycholesterol derivatives (Chemical and Pharmaceutical Bulletin, Vol. 21, pp. 2783-2785, 1973). In this method, 24R-form and the 24S-form thereof also are produced as a substantially equivalent mixture.

Cis-dihydroxylation with osmium tetroxide has been known as a method for converting an olefinic double bond into a vic-diol, and recently there has been developed an asymmetric dihydroxylation method which is capable of catalytically converting an olefinic double bond into a vic-diol with high stereoselectivity in the presence of an osmium complex using a cinchona alkaloid derivative as ligand (Japanese Patent Application corresponding to PCT application Laid-Open (KOHYO) Nos. 3-503885, 5-509297, 6-50034 and 7-500323, and Journal of Organic Chemistry, Vol.57, pp. 2768-2771, 1992).

An attempt of application of the above catalytic asymmetric dihydroxylation method to a desmosterol derivative is disclosed in Japanese Patent Application Laid-Open (KOKAI) No. 7-233192. According to this Japanese KOKAI, an osmium complex using a cinchona alkaloid derivative as ligand is acted to desmosteryl benzoate in a tert-butanol/water two-component solvent system at 0°C for 20 hours to produce (24R)-24,25-dihydroxycholesteryl benzoate in an overall yield of 89% with an R-diastereomer excess percentage of 90%.

However, desmosterol has double bonds not only at the 24-position but also at the 5-position. It is known that for the double bond at the 5-position there is a spatially difficulty of access of reagents due to the presence of methyl groups at the 18-and 19-positions, so that the reactivity of the double bond at the 5-position is lower than that of the double bond at the 24-position but is not negligibly low. The fact that the reactivity of the double bond at the 5-position can never be neglected is evident by a report that cholestane-3 β,5α,6α-triol could be obtained in a maximum yield of 74% by catalytically dihydroxylating cholesterol having the same structure as desmosterol except for the absence of double bond at the 24-position in the presence of osmium complexes having various tertiary amines as ligands and a co-oxidant at room temperature for 24 hours (Journal of Organic Chemistry, Vol. 55, pp. 766-768, 1990).

In the double bond at the 5-position of desmosterol derivatives by subjecting to the said catalytic asymmetric dihydroxylation reaction, a by-product may be produced. Therefore, desmosterol derivatives having double bonds not only in the side chain to be reacted but also in the steroid nucleus can not be deemed the best material for producing the (24R)-24,25-dihydroxysteroid compounds.

It is apparent from the above explanation that in case of using a steroid compound capable of being subject to catalytic asymmetric dihydroxylation as a starting material for the preparation of (24R)-24,25-dihydroxysteroid compounds, it is preferable to select steroid compounds having double bond only at the 24-position of the side chain.

Generally, catalytic asymmetric dihydroxylation of steroid compounds is accompanied by the difficulties which have been unknown in the art. Most of the steroid compounds are almost insoluble in the tert-butanol/water heterogeneous solvent system which is suited for the catalytic asymmetric dihydroxylation, so that when using such steroid compounds the reaction scarcely proceeds. As a solution to this problem, it is suggested that by adding an appropriate organic solvent, the steroid substrate is dissolved in the reaction solvent system.

However, since the addition of such an organic solvent could cause adverse effects such as disturbance of the heterogeneous solvent system comprising mainly of tert-butanol and water or impairment of the stability and reactivity of the osmium complex, it is hardly possible to attain the catalytic asymmetric dihydroxylation in the desired way. Therefore, circumspection is required in selecting the organic solvent to be used.

As a result of the present inventors' earnest studies on the subject matter, it has been found that by carrying out catalytic asymmetric dihydroxylation reaction on steroid compounds having carbon-carbon double bond in the side chain in the presence of an osmium complex having a hydroquinidine derivative as ligand in a heterogeneous solvent system prepared by adding at least one organic solvent selected from the group consisting of tetrahydrofuran, acetonitrile, acetone, 1,4-dioxane, N,N-dimethylformamide and N,N-dimethylacetamide to a tert-butanol/water two-component solvent system, it is possible to produce (24R)-24,25-dihydroxysteroid compounds in a high yield. The present invention has been attained on the basis of this finding.

The object of the present invention is to provide a process for producing (24R)-24,25-dihydroxysteroid compounds in a high yield with a high-level diastereomer excess percentage by carrying out catalytic asymmetric dihydroxylation reaction in a heterogeneous solvent system prepared by adding an organic solvent to a tert-butanol/water two-component solvent system and having no possibility of impairing the stability and reactivity of the osmium complex present in the reaction system by using a substrate having the structure defined in the present invention and showing sufficient solubility in the said solvent system.

To accomplish the aim, in an aspect of the present invention, there is provided a process for producing (24R)-24,25-dihydroxysteroid compounds represented by the following formula (I), which comprises carrying out catalytic asymmetric dihydroxylation reaction of steroid compounds having a carbon-carbon double bond in the side chain and represented by the following formula (II) in the presence of an osmium complex having a hydroquinidine derivative as ligand by using a heterogeneous solvent system prepared by adding at least one organic solvent selected from the group consisting of tetrahydrofuran, acetonitrile, acetone, 1,4-dioxane, N,N-dimethylformamide and N,N-dimethylacetamide to a tert-butanol/water two-component solvent system: 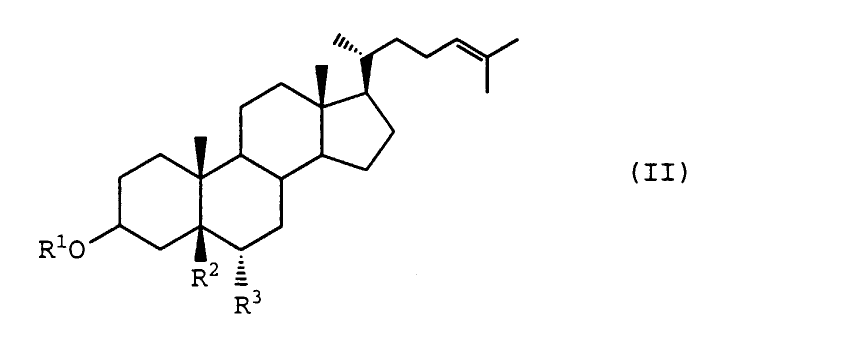 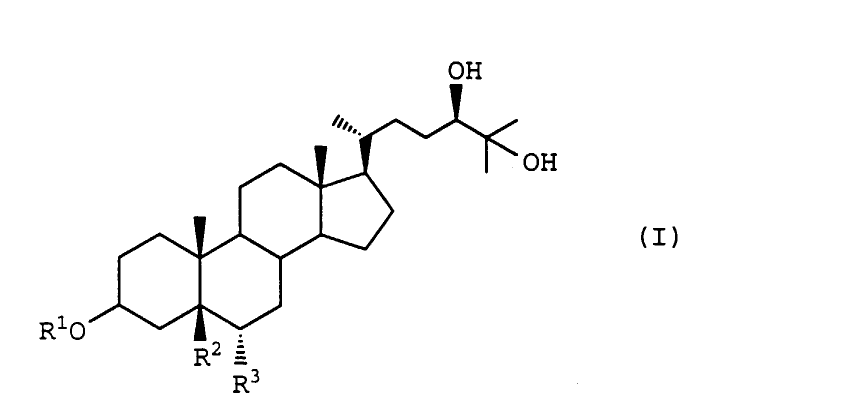 wherein R¹ represents a hydrogen atom, an acetyl group, a benzoyl group, a 2'-tetrahydropyranyl group, a 2'-methoxyethoxymethyl group, methanesulfonyl group or p-toluenesulfonyl group; R² represents either a hydrogen atom or, combined with R³, a carbon-carbon bond; R³ represents either R¹O or, combined with R², a carbon-carbon bond.

The starting material used in the process of the present invention is steroid compounds having a carbon-carbon double bond in the side chain represented by the above-shown formula (II) (hereinafter referred to as "substrate"). R¹ in the formula (II) represents a hydrogen atom, a hydroxyl-protecting group or a hydroxyl-activating group. The hydroxyl-protecting groups in R¹ include alkyl groups having 1 to 4 carbon atoms, aliphatic acyl groups having 1 to 4 carbon atoms, and aromatic acyl groups. Among them, 2-tetrahydropyranyl group and 2'-methoxyethoxymethyl group are particularly preferred. The hydroxyl-activating groups in R¹ include aliphatic sulfonyl groups having 1 to 2 carbon atoms and aromatic sulfonyl groups. Among them, methanesulfonyl group and p-toluenesulfonyl group are particularly preferred.

An example of the said substrate is 5β-cholest-24-ene-3α,6α-diol in case where R¹ is a hydrogen atom. Considering that the substrate is later converted into (24R)-24,25-dihydroxycholesterol, it is preferable that the hydroxyl groups at the 3α- and 6α-positions be protected or activated before carrying out the process of the present invention.

In case where R¹ is a hydroxyl-protecting group, 5β-cholest-24-ene-3α,6α-ditetrahydropyranyl ether and 5β-cholest-24-ene-3α,6α-dimethoxyethoxymethyl ether can be mentioned as examples of the said substrate. By using these protective groups, the protecting reaction and the deprotecting reaction can be easily carried out and also a high yield is achieved. Further, the presence of a protected hydroxyl group at the 3α-and 6α-positions facilitates the dissolution of the substrate in the reaction solvent system for the asymmetric dihydroxylation to allow smooth proceeding of the reaction in the process of the present invention.

In case where R¹ is a hydroxyl-activating group, 5β-cholest-24-ene-3α,6α-dimethanesulonate, 5β-cholest-24-ene-3α,6α-di(p-toluene)sulfonate and the like can be exemplified as the said substrate. These substrates are converted into (24R)-5β-cholestane-24,25-diol-3α,6α-dimethanesulfonate or p-toluenesulfonate according to the process of the present invention, then reacted with a base such as potassium acetate in an N,N-dimethylformamide/water mixed solvent and saponified, to produce (24R)-24,25-dihydroxycholesterol.

R² in the formula (II) represents a hydrogen atom, or R² may combine with R³ to form a carbon-carbon single bond. In case where R² is a hydrogen atom, the above-mentioned steroid compound can, for instance, be used as the substrate. In case where R² and R³ are combined to form a carbon-carbon single bond, desmosterol and its derivatives in which the hydroxyl group at the 3β-position has been modified by the said protective group, such as desmosteryl acetate, desmosteryl benzoate, tetrahydropyranyl and methoxyethoxymethyl ethers of desmosterol, and the like can be mentioned as examples of the said substrate.

In the process of the present invention, there are used a heterogeneous solvent system prepared by adding an organic solvent to a tert-butanol/water two-component solvent system and the said heterogeneous solvent system having no possibility of impairing the stability and reactivity of the osmium complex, which has a hydroquinidine derivative as ligand. The organic solvent material is used in such an amount and under such a condition that it does not disturb the tert-butanol/water two-component heterogeneous solvent system and it does not give any adverse effect on the stability and reactivity of the osmium complex. Further, potassium hexacyanoferrate (III), potassium carbonate and methanesulfonamide are used to allow smooth proceeding of catalytic dihydroxylation. Without these adjuncts, the reaction may fail to proceed smoothly.

As the above organic solvent, there is usually used at least one organic solvent selected from the group consisting of tetrahydrofuran, acetonitrile, acetone, 1,4-dioxane, N,N-dimethylformamide and N,N-dimethylacetamide. Among these solvents, tetrahydrofuran and acetonitrile are preferred.

A preferred example of the said hydroquinidine derivative is 1,4-phthalazinediyl diether of hydroquinidine. When a hydroquinidine derivative is used, the 24R-form of 24,25-dihydroxy steroid compound is produced, and when a hydroquinine derivative is used, the 24S-form thereof is produced.

The organic solvent is used in an amount of usually 0.1 to 10 parts by volume, preferably 0.5 to 5 parts by volume based on one part by volume of tert-butanol. The amount of water is not restricted as far as it allows formation of heterogeneous layers in the reaction, and usually it is in the range of 0.8 to 1.2 parts by volume, preferably 0.9 to 1.1 parts by volume based on one part by volume of tert-butanol.

When the amount of the organic solvent used is less than 0.1 part by volume or more than 10 parts by volume based on one part by volume of tert-butanol, the desired heterogeneous solvent system may not be prepared and the reaction may not proceed.

Also, when the amount of water used is less than 0.8 parts by volume or more than 1.2 parts by volume based on one part by volume of tert-butanol, the desired heterogeneous solvent system may not be prepared and the reaction may not proceed.

The amount of the osmium complex and other components used are as follows. The osmium complex having a hydroquinidine derivative as ligand is used in an amount of usually 1 to 10 µmol, preferably 1.5 to 5 µmol based on one mmol of the compound represented by the formula (II). When the amount of the osmium complex used is less than 1 µmol or more than 10 µmol based on one mmol of the compound represented by the formula (II), the reaction may not be complete and the excess percentage of the desired diastereomer may be low.

Potassium hexacyanoferrate (III) is used in an amount of usually 1 to 6 mmol, preferably 2 to 5 mmol based on one mmol of the compound represented by the formula (II). When the amount of potassium hexacyanoferrate (III) used is less than 1 mmol based on one mmol of the compound represented by the formula (II), the reaction may stop before reaching completion. When the amount of potassium hexacyanoferrate (III) used is more than 6 mmol based on one mmol of the compound represented by the formula (II), the production cost may be expensive and work-up may be troublesome.

Methanesulfonamide is used in an amount of usually 0.1 to 3 mmol, preferably 0.5 to 2 mmol based on one mmol of the compound represented by the formula (II). When the amount of methanesulfonamide used is less than 0.1 mmol based on one mmol of the compound represented by the formula (II), the excess percentage of the diastereomer may be low. When the amount of methanesulfonamide used is more than 3 mmol based on one mmol of the compound represented by the formula (II), the production cost may be expensive and work-up may be troublesome.

Potassium carbonate is used in an amount of usually 0.01 to 1 part by weight, preferably 0.05 to 0.8 parts by weight based on one part by weight of water. When the amount of potassium carbonate used is less than 0.01 part by weight or more than one part by weight based on one part by weight of water, the reaction may not be complete and the excess percentage of the diastereomer may be low.

The present invention can be conducted according to any of the following procedures (1) to (3), but in any procedure the reaction is conducted in a heterogeneous solvent system.
(1) tert-Butanol, water and an organic solvent(s) are mixed, the prescribed amounts of potassium hexacyanoferrate (III), potassium carbonate, 1,4-phthalazinediyl diether of hydroquinidine, potassium osmate (VI) dihydrate and methanesulfonamide are added to the prepared solvent system, the temperature of the mixture is adjusted to the reaction temperature with stirring, the reaction substrate dissolved in an organic solvent is added to the mixture finally, and the reaction mixture is stirred until the reaction is complete.
(2) The prescribed amounts of potassium hexacyanoferrate (III), potassium carbonate, 1,4-phthalazinediyl diether of hydroquinidine, potassium osmate (VI) dihydrate and methanesulfonamide are added to a mixture of tert-butanol and water, the temperature of the mixture is adjusted to the reaction temperature with stirring, the reaction substrate dissolved in an organic solvent is added to the mixture finally, and the reaction mixture is stirred until the reaction is complete.
(3) The prescribed amounts of potassium hexacyanoferrate (III), potassium carbonate, 1,4-phthalazinediyl diether of hydroquinidine, potassium osmate (VI) dihydrate and methanesulfonamide are added to a mixture of tert-butanol, water and an organic solvent(s), the temperature of the mixture is adjusted to the reaction temperature with stirring, the solid reaction substrate is added to the mixture finally, and the reaction mixture is stirred until the reaction is complete.

In any of these processes, the reaction is carried out in such a manner that the reaction substrate will be uniformly dissolved principally in the organic phase.

In the present invention, the reaction temperature is usually in the range from -20°C to 50°C, preferably from -10°C to 30°C, and the reaction time is usually in the range from 6 to 120 hours, preferably from 10 to 60 hours.

When the reaction temperature is below -20°C, the reaction may not be complete. When the reaction temperature is above 50°C, the yield of the objective product may be low and a side reaction may occur.

When the reaction time is less than 6 hours, the reaction may not be complete. When the reaction time is more than 120 hours, the yield of the objective product may be low.

The reaction product in the present invention is purified in the following way. A reducing agent such as sodium sulfite is added to the reaction mixture to inactivate the excess co-oxidant, and then a solvent such as dichloromethane, ethyl acetate or the like is added to extract the organic matter. If necessary, the extracted organic matter is washed with a dilute acidic and/or alkaline aqueous solutions, dehydrated with anhydrous magnesium sulfate or the like, concentrated, and then purified by column chromatography, recrystallization or other means.

The yield of the objective product purified in the manner described above is not less than 90 %, and the excess percentage of the desired diastereomer is not less than 90 %.

According to the present invention described above, it is possible to obtain (24R)-24,25-dihydroxysteroid compounds in a high yield with a high-level diastereomer excess percentage by carrying out catalytic asymmetric dihydroxylation of substrates in a heterogeneous solvent system prepared by adding an organic solvent to a tert-butanol/water two-component solvent system and having no liability to impair the stability and reactivity of the osmium complex present in the reaction system.

### EXAMPLES

The present invention is further illustrated by the following examples, but these examples are merely intended to be illustrative and not to be construed as limiting the scope of the invention.

### Example 1

### <Synthesis of (24R)-5β-cholest-24-ene-24,25-diol-3α,6α-di(2'-tetrahydropyranyl)ether>

A 2.5 ml of tert-butanol, 2.5 ml of water, 3.85 ml (4.94 µmol) of 1,4-phthalazinediyl diether of hydroquinidine, 0.46 mg (1.24 µmol) of potassium osmate (VI) dihydrate, 487.6 mg (1.48 mmol) of potassium hexacyanoferrate (III), 204.8 mg (1.48 mmol) of potassium carbonate and 48.2 mg (0.507 mmol) of methanesulfonamide were mixed, stirred at room temperature for 30 minutes and then cooled to 0°C.

208.1 mg (0.365 mmol) of 5β-cholest-24-ene-3α,6α-di(2'-tetrahydropyranyl)ether dissolved in 2.0 ml of tetrahydrofuran was added to the cooled solution. After stirring at 0°C for 24 hours, 750 mg (5.95 mmol) of anhydrous sodium sulfite was added and the reaction temperature of the solution was returned to room temperature with stirring and further stirred for an hour. This reaction solution was extracted thrice with 10 ml of dichloromethane each time, the combined dichloromethane layers were dehydrated with anhydrous magnesium sulfate and then dichloromethane was distilled away to obtain a yellow syrupy crude product.

This crude product was separated by silica gel column chromatography using a hexane/ethyl acetate (1/1) mixed solvent and the separated solution was concentrated to obtain a colorless syrupy product. This product was further dried *in vacuo* to give 204.2 mg (0.338 mmol) of a foamy 5β-chorest-24-ene-24,25-diol-3α,6α-di(2'-tetrahydropyranyl)ether in a 92.6 mmol% yield.

The whole amount of the said product was benzoylated with benzoyl chloride at room temperature in the presence of 4-dimethylaminopyridine in dichloromethane and stirred at 80°C for one hour in the presence of p-toluenesufonic acid in ethanol to remove tetrahydropyranyl groups, then converting the product into (24R)-5β-cholestane-3α,6α,25-triol-24-benzoate. The diastereomer ratio of the said converted product was determined by high-performance liquid chromatography (column: NovaPAK Silica, 7.5 cm; solvent: hexane/dichloromethane/2-propanol = 50/50/8, 0.6 ml/min). The ratio of 24R-form to 24S-form was 97:3. The 24R-diastereomer excess percentage was 94%.

### Example 2

### <Synthesis of (24R) -5β-cholestane-24,25-diol-3α,6α-di(p-toluene)sulfonate>

A 25 ml of tert-butanol, 25 ml of water, 75 ml of tetrahydrofuran, 147 mg (0.189 mmol) of 1,4-phthalazinediyl diether of hydroquinidine, 13.8 mg (37.5 µmol) of potassium osmate(VI) dihydrate, 21.68 g (65.8 mmol) of potassium hexacyanoferrate (III), 7.80 g (56.4 mmol) of potassium carbonate and 1.80 g (18.9 mmol) of methanesulfonamide were mixed, stirred at room temperature for 30 minutes and then cooled to 0°C.

13.5 g (19.0 mmol) of 5β-cholest-24-ene-3α,6α-di(p-toluene)sulfonate dissolved in 20 ml of tetrahydrofuran was added to the cooled solution. After stirring at 0°C for 40 hours, 33.2 g (263 mmol) of sodium sulfite and 25 ml of water were added to the reaction mixture and the reaction temperature of the solution was returned to the room temperature and the mixture was stirred for an hour. The mixture was then allowed to stand and separated into an organic layer and an aqueous layer, and the aqueous layer was extracted twice with 30 ml of ethyl acetate each time.

The extract and the organic layers were combined and dehydrated with anhydrous magnesium sulfate, and the solvent was removed away to obtain a yellow syrupy crude product. This crude product was separated and purified by silica gel column chromatography using a hexane/ethyl acetate (4/1) mixed solvent, then concentrated and dried in vacuo to obtain 13.5 g (28.1 mmol) of an amorphous foam in a 95.7 mol% yield. The NMR analysis of the product gave the following data:
1H-NMR spectrum (CDCl₃, 60 MHz) δppm: 0.577 (3H, s), 0.783 (3H, s), 0.883 (3H, d), 1.12 (3H, s), 1.17 (3H, s), 2.43 (6H, s), 3.30 (1H, m), 7.21-7.81 (8H, m).

The whole amount of the product was subjected to the reaction with potassium acetate, saponification and benzoylation reaction successively and thereby converted into 24,25-dihydroxycholesterol-3,24-dibenzoate, and the diastereomer ratio was determined by high-performance liquid chromatography (column: NovaPAK Silica, 7.5 cm; solvent:
hexane/dichloromethane/2-propanol = 70/30/0.25, 0.6 ml/min). The ratio of 24R-form to 24S-form was 97:3, and the R-diastereomer excess percentage was 94%.

### Example 3

The reaction procedure of Example 2 was repeated except for the absence of tetrahydrofuran in the solution prepared and cooled. 13.5 g (19.0 mmol) of 5β-cholest-24-ene-3α,6α-di(p-toluene)sulfonate dissolved in 95 ml of tetrahydrofurn was added to the cooled solution. Although the substrate was precipitated in a syrupy form in the early stage of the reaction, it was perfectly dissolved in an hour. Thereafter the same operations according to Example 2 were repeated to recover the reaction product and convert it into 24,25-dihydroxycholesterol-3,24-dibenzoate, and the diastereomer ratio was determined. The ratio of 24R-form to 24S-form was 97:3. The R-diastereomer excess percentage was 94%.

### Example 4

### <Synthesis of (24R) -5β-cholestane-24,25-diol-3α,6α-ditosylate>

A 20 ml of tert-butanol, 20 ml of water, 25 ml of acetonitrile, 30.9 mg (39.7 µmol) of 1,4-phthalazinediyl diether of hydroquinidine, 2.91 mg (7.91 µmol) of potassium osmate(VI) dihydrate, 5.27 g (16.0 mmol) of potassium hexacyanoferrate(III), 1.65 g (11.9 mmol) of potassium carbonate and 0.571 g (6.00 mmol) of methanesulfonamide were mixed and stirred at room temperature for 30 minutes.

A solution of 2.84 g (4.00 mmol) of 5β-cholest-24-ene-3α,6α-ditosylate dissolved in 3 ml of acetonitrile was added to the above solution and the solution was stirred at 20°C for 20 hours. Then, 6.00 g (47.6 mmol) of sodium sulfite and 10 ml of water were added to the reaction mixture and the mixture was stirred at room temperature for an hour. The mixture was then allowed to stand and separated into an organic layer and an aqueous layer, and the residual organic matter in the aqueous layer was extracted twice with 20 ml of ethyl acetate each time.

The extract and the organic layers were combined and dehydrated with anhydrous magnesium sulfate, and the solvent was removed away to give a yellow syrupy crude product. This crude product was separated and purified by silica gel column chromatography using a hexane/ethyl acetate (3/1) mixed solvent, then concentrated and dried in vacuo to obtain 2.89 g (3.88 mmol) of an amorphous foam in a 97.0 mol% yield. The product was converted into 24,25-dihydroxycholesterol-3,24-dibenzoate by the same operations as in Example 2, and the diastereomer ratio was measured. The ratio of 24R-form to 24S-form was 98:2, and the R-diastereomer excess percentage was 96%.

### Comparative Example 1

The reaction procedure of Example 1 was repeated except that 5β-cholest-24-ene-3α,6α-di(2'-tetrahydropyranyl)ether was added without being dissolved in tetrahydrofuran but in the form as it is (solid). TLC observation of a dichloromethane solution of the product showed that only a slight amount of the objective substance was produced. The main part of the product consisted of the unreacted substrate.

### Comparative Example 2

The reaction procedure of Example 1 was repeated except that hexane was used in place of tetrahydrofuran. The substrate remained substantially unreacted.

### Comparative Example 3

The reaction procedure of Example 2 was carried out except that no tetrahydrofuran was contained in the solvent system and 13.5 g (19.0 mmol) of powdery 5β-cholest-24-ene-3α,6α-di(p-toluene)sulfonate was added to the cooled solution. The substrate solidified into a gummy form and could not be dissolved. After stirring for 40 hours, the organic layer was extracted with ethyl acetate and its contents were examined by TLC. There was produced only a slight amount of the objective substance, and the main part of the product consisted of the unreacted starting material.

## Claims

1. A process for producing a (24R)-24,25-dihydroxysteroid compound of following formula (I), which comprises carrying out a catalytic asymmetric dihydroxylation reaction on a steroid compound having a carbon-carbon double bond in the side chain of following formula (II) in the presence of an osmium complex having a hydroquinidine derivative as ligand, potassium hexacyanoferrate (III) as a co-oxidant, potassium carbonate and methanesulfonamide, by using a heterogeneous solvent system prepared by adding at least one organic solvent selected from tetrahydrofuran, acetonitrile, acetone, 1,4-dioxane, N,N-dimethylformamide and N,N-dimethylacetamide to a tert-butanol/water two-component solvent system having a volume ratio of 1:0.8-1.2 in an amount of 0.1-10 parts by volume based on one part by volume of tert-butanol: 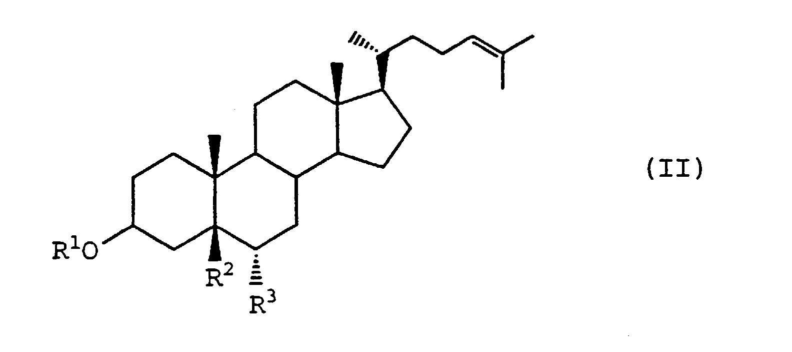 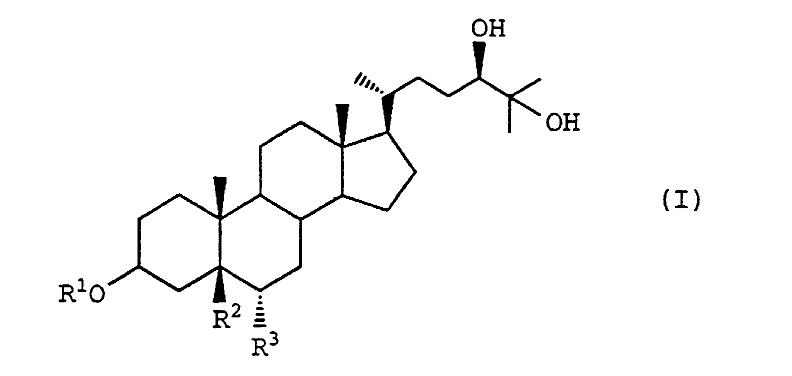 wherein R¹ represents a hydrogen atom, an acetyl group, a benzoyl group, a 2'-tetrahydropyranyl group, a 2'-methoxyethoxyethyl group, a methanesulfonyl group or a p-toluenesulfonyl group; R² represents a hydrogen atom; and R³ represents R¹O wherein R¹ is as defined above and may be the same or different from the above R¹ group, or R² and R³ together form a carbon-carbon bond.

2. A process according to claim 1, wherein the hydroquinidine derivative is the 1,4-phthalazinediyl diether of hydroquinidine.

3. A process according to claim 1 or 2, wherein the osmium complex is used in an amount of 1 to 10 µmol based on one mol of the compound of formula (II).

4. A process according to any one of the preceding claims, wherein the potassium hexacyanoferrate (III) is used in an amount of 1 to 6 mmol based on one mmol of the compound of formula (II).

5. A process according to any one of the preceding claims, wherein the methanesulfonamide is used in an amount of 0.1 to 3 mmol based on one mmol of the compound of formula (II).

6. A process according to any one of the preceding claims, wherein the potassium carbonate is used in an amount of 0.01 to 1 part by weight based on one part by weight of water.

7. A process according to any one of the preceding claims, wherein the reaction is carried out at a temperature of from -20°C to 50°C.

8. A process according to any one of the preceding claims, wherein the reaction is carried out for a period of 6 to 120 hours.

9. A process according to any one of the preceding claims, wherein the reaction is carried out by mixing tert-butanol, water and one or more of said organic solvents, adding potassium hexacyanoferrate (III), potassium carbonate, a hydroquinidine derivative, potassium osmate (VI) dihydrate and methanesulfonamide to the mixture, adjusting the temperature of the mixture to the reaction temperature with stirring, adding a compound of formula (II) dissolved in an organic solvent, and stirring the mixture until the reaction is complete.

10. A process according to any one of claims 1 to 8, wherein the reaction is carried out by mixing tert-butanol and water, adding potassium hexacyanoferrate (III), potassium carbonate, a hydroquinidine derivative, potassium osmate (VI) dihydrate and methanesulfonamide to the mixture, adjusting the temperature of the mixture to the reaction temperature with stirring, adding a compound of formula (II) dissolved in said organic solvent, and stirring the mixture until the reaction is complete.

11. A process according to any one of claims 1 to 8, wherein the reaction is carried out by mixing tert-butanol, water and one or more of said organic solvents, adding potassium hexacyanoferrate (III), potassium carbonate, a hydroquinidine derivative, potassium osmate (VI) dihydrate and methanesulfonamide to the mixture, adjusting the temperature of the mixture to the reaction temperature with stirring, adding a solid compound of formula (II), and stirring the mixture until the reaction is complete.

12. A process according to any one of claims 9 to 11, wherein after the completion of the reaction, a reducing agent is added to the reaction mixture to inactivate the excess co-oxidant and the organic matter is extracted to purify the reaction product.

13. A process according to claim 12, wherein the reducing agent is sodium sulfite.

14. A process according to claim 12 or 13, wherein the solvent used for extracting the organic matter is dichloromethane or ethyl acetate.
